# EUROPEAN PATENT APPLICATION

(11) **EP 4 331 582 A1**
(43) Date of publication of application: **06.03.2024**
(21) Application number: 22795412.0
(22) Date of filing: 25.03.2022
(51) Int. Cl.: A61K 31/4172, A23L 33/175, A61P 35/00, A61P 37/04, A61P 43/00

(54) **COMPOSITION FOR STIMULATING IMMUNE CELL METABOLISM**

(30) Priority: 26.04.2021 JP 2021074323
(71) Applicant: Suntory Holdings Limited, Osaka 530-8203 (JP)
(72) Inventor: KATSUBE, Makoto, Soraku-gun, Kyoto 619-0284 (JP); WATANABE, Hiroshi, Soraku-gun, Kyoto 619-0284 (JP); OZAWA, Mariko, Soraku-gun, Kyoto 619-0284 (JP)
(74) Representative: Vossius & Partner Patentanwälte Rechtsanwälte mbB
(86) International application number: PCT/JP2022/014256
(87) International publication number: WO 2022/230494

(57) **Abstract**

The present invention aims to provide a composition for promoting immune cell metabolism, a composition for activating immunity, a method of promoting immune cell metabolism, and a method of activating immunity. The present invention relates to a composition for promoting immune cell metabolism, which contains L-ergothioneine or a salt thereof as an active ingredient.

## Description

### TECHNICAL FIELD

The present invention relates to a composition for promoting immune cell metabolism. The present invention also relates to a composition for activating immunity. The present invention further relates to a method of promoting immune cell metabolism, a method of activating immunity, and the like.

### BACKGROUND ART

Immune cells (also referred to as "immunocompetent cells") are present in various types and known to exert immune function (innate immunity and acquired immunity) while interacting with each other. For example, leukocytes are generally classified into the following five types of immunocompetent cells involved in biological defense: monocytes (macrophages), lymphocytes, neutrophils, basophils, and eosinophils. These cells are hematopoietic stem cell-derived cells that play a role in eliminating foreign substances such as bacteria and viruses that have entered the body from the outside and eliminating tumor cells and cells that have completed their roles. Peripheral blood mononuclear cells (PBMCs) do not remain in the lymphatic system, spleen, liver, or bone marrow. Instead, these cells are found in peripheral blood circulating throughout the body and consist of four cell types including T cells, B cells, natural killer cells, and monocytes.

The immune function of these immune cells is known to decline with aging. Presumedly, the main cause is that the thymus, which is responsible for the production of T cells, and the spleen, which is rich in lymphocytes, atrophy faster than other organs with aging.

Ergothioneine is an amino acid found in mushrooms and the like. L-ergothioneine is present in nature. According to Patent Literature 1, the peritesticular fat weight and the triglyceride (neutral fat) level in plasma were lower in mice orally administered with ergothioneine than in mice not orally administered with ergothioneine.

Patent Literature 2 discloses an agent for promoting the production of immune response activating cytokines, which contains, as active ingredients, ergothioneine and any one Toll-like receptor ligand selected from lipopolysaccharide (LPS), Pam2CSK4, synthetic triacylated lipoprotein, and imidazoquinolines. In the absence of the TLR ligand, none of IL-6, IL-12, IL-1β, and IL-10 was detected in any sample with an ergothioneine concentration of either 0 or 10 mM. In other words, according to the disclosure, when TLR was not stimulated, none of IL-6, IL-12, IL-1β, and IL-10 was detected, regardless of the presence or absence of ergothioneine (claim 1 and elsewhere, and paragraph [0068]). Thus, an agent for promoting the production of immune response activating cytokines has been known which contains, as active ingredients, ergothioneine and a specific Toll-like receptor ligand. However, it has not been known that ergothioneine itself has an action to promote the production of immune response activating cytokines.

Patent Literature 3 discloses a method of protecting mitochondria from damage caused by radiation, free radicals, and reactive oxygen species. The method includes parenteral, topical, transmucosal, pulmonary, or transdermal administration of a composition containing L-ergothioneine to mitochondria (claim 1, claim 8, and elsewhere). However, the literature is completely silent about activation of mitochondrial function by L-ergothioneine, and there is no description or suggestion about promotion of immune cell metabolism.

### CITATION LIST

### - Patent Literature

Patent Literature 1: JP 2011-102286 A
Patent Literature 2: JP 6121597 B
Patent Literature 3: JP 2001-513760 A

### SUMMARY OF INVENTION

### - Technical Problem

It has not been known that L-ergothioneine or a salt thereof has an action to promote immune cell metabolism.

The present invention aims to provide a composition for promoting immune cell metabolism. The present invention also aims to provide a composition for activating immunity. The present invention still also aims to provide a method of promoting immune cell metabolism and a method of activating immunity.

### - Solution to Problem

As a result of extensive studies to solve the problem described above, the present inventors found that L-ergothioneine has an action to promote immune cell metabolism.

Specifically, the present invention relates to, but is not limited to, a composition for promoting immune cell metabolism, a composition for activating immunity, and the like described below.
(1) A composition for promoting immune cell metabolism, containing L-ergothioneine or a salt thereof as an active ingredient.
(2) The composition according to (1) above, wherein the composition promotes immune cell metabolism by activating mitochondrial function.
(3) A composition for activating immunity, containing the composition according to (1) or (2) above.
(4) The composition according to any one of (1) to (3) above, wherein the composition is an oral composition.
(5) The composition according to any one of (1) to (4) above, wherein the composition is a food or beverage.
(6) The composition according to any one of (1) to (5) above, wherein an amount of L-ergothioneine or a salt thereof in the composition per adult daily intake is 2 to 50 mg in terms of L-ergothioneine.
(7) A method of promoting immune cell metabolism, including administering L-ergothioneine or a salt thereof.
(8) A method of activating immunity, including administering L-ergothioneine or a salt thereof.
(9) Use of L-ergothioneine or a salt thereof to promote immune cell metabolism.
(10) Use of L-ergothioneine or a salt thereof to activate immunity.

### - Advantageous Effects of Invention

The present invention can provide a composition for promoting immune cell metabolism. The present invention can also provide a composition for activating immunity, a method of promoting immune cell metabolism, and a method of activating immunity.

### BRIEF DESCRIPTION OF DRAWINGS

FIG. 1A is a graph showing the impact of ergothioneine on the oxygen consumption rate (OCR) related to basal respiration. FIG. 1B is a graph showing the impact of ergothioneine on the OCR related to ATP-related respiration. FIG. 1C is a graph showing the impact of ergothioneine on the OCR related to maximum respiration.
FIG. 2 is a graph showing the impact of ergothioneine on the oxygen consumption rate (OCR) of human PBMCs over time.

### DESCRIPTION OF EMBODIMENTS

The composition for promoting immune cell metabolism of the present invention contains L-ergothioneine or a salt thereof as an active ingredient. Hereinafter, the composition for promoting immune cell metabolism of the present invention is sometimes referred to as "the composition according to the first embodiment of the present invention".

L-ergothioneine is one of the amino acids.

The salt of L-ergothioneine is not limited as long as it is a pharmacologically acceptable salt or a dietary acceptable salt, and it may be either an acid salt or a basic salt. Examples of the acid salt include inorganic acid salts such as hydrochloride, sulfate, nitrate, and phosphate, and organic acid salts such as acetate, citrate, maleate, malate, oxalate, lactate, succinate, fumarate, and propionate. Examples of the basic salt include alkali metal salts such as sodium salt and potassium salt, and alkaline earth metal salts such as calcium salt and magnesium salt.

L-ergothioneine or a salt thereof that can be used may be a chemically synthesized product or a purified extract of a natural product. L-ergothioneine is abundantly present in golden/yellow oyster mushrooms (binomial name: *Pleurotus cornucopiae* var. *citrinopileatus*) belonging to the genus *Pleurotus* of the family Pleurotaceae. L-ergothioneine is also present in mushrooms such as common mushrooms (binomial name: *Agaricus bisporus*) including white mushroom, cremini mushroom, and portabella mushroom; grey oyster mushroom (binomial name: *Pleurotus ostreatus)*, shiitake (binomial name: *Lentinula edodes*), hen-of-the-wood (binomial name: *Grifola Frondosa*), reishi mushroom (binomial name: *Ganoderma lucidum*), lion's mane mushroom (binomial name: *Hericium erinaceus*), Yanagi-matsutake (binomial name: *Agrocybe aegerita*), girolle (binomial name: Cantharellus cibarius), porcini (binomial name: *Boletus edulis*), and common morel (binomial name: *Morchella esculenta*). When L-ergothioneine is obtained from a natural product, preferably, it is extracted from a golden/yellow oyster mushroom, for example. L-ergothioneine or a salt thereof can also be produced by microbial fermentation. An extract containing L-ergothioneine or a salt thereof produced by microbial fermentation or a purified product thereof may be used. L-ergothioneine can be extracted and purified from natural products by known methods. L-ergothioneine or a salt thereof may be in an isolated form.

L-ergothioneine or a salt thereof is present in natural products and food and beverages, and it is a compound that has been consumed. Thus, continuous ingestion of L-ergothioneine or a salt thereof, for example, is less likely to cause problems in terms of safety.

As shown in Examples (described later), adding L-ergothioneine increased the oxygen consumption rate (OCR) of peripheral blood mononuclear cells (PBMCs) and significantly increased mitochondrial metabolism, compared to no addition.

Mitochondria are subcellular organelles present in all oxygen-utilizing organisms in which energy is generated in the form of adenosine triphosphate (ATP) and oxygen is reduced to water. An increase in mitochondrial metabolism in peripheral blood mononuclear cells (PBMCs) is indicated by an increase in mitochondrial basal respiration and maximum respiration, an increase in amount of mitochondria ATP production, and the like. An increase in mitochondrial basal respiration and maximum respiration and an increase in amount of ATP production can be rephrased as an increase in energy production due to activation of mitochondrial function. These can be used as indices for promotion of immune cell metabolism.

Thus, L-ergothioneine can be used as an active ingredient for promoting immune cell metabolism. The cellular oxygen consumption rate (OCR), mitochondrial basal respiration, maximum respiration, and amount of ATP production can be measured by known methods. For example, as shown in Examples (described later), an XFe96 flux analyzer (available from Agilent Technologies) can be used for measurement.

Preferably, the composition of the present invention promotes immune cell metabolism by activating mitochondrial function in immune cells. As described above, the use of L-ergothioneine significantly increased the mitochondrial basal respiration, ATP production, and maximum respiration of peripheral blood mononuclear cells (PBMCs) and significantly increased mitochondrial metabolism, compared to no use. This shows the use of L-ergothioneine activates mitochondrial function. In one embodiment, the composition of the present invention can be used to promote immune cell metabolism by activating mitochondrial function.

In the present invention, preferably, the immune cells are at least one type selected from T cells, B cells, natural killer cells, and monocytes. It is because peripheral blood mononuclear cells (PBMCs) do not remain in the lymphatic system, spleen, liver, or bone marrow but are found in the peripheral blood circulating throughout the body, and consist of four cell types including T cells, B cells, natural killer cells, and monocytes. Preferably, the composition of the present invention promotes metabolism of at least one type of immune cells selected from T cells, B cells, natural killer cells, and monocytes by activating mitochondrial function in peripheral blood mononuclear cells (PBMCs).

L-ergothioneine or a salt thereof has an action to promote immune cell metabolism and an action to activate mitochondrial function in immune cells, so that L-ergothioneine or a salt thereof has an action to activate immunity. L-ergothioneine or a salt thereof can be used as an active ingredient for activating immunity. In one embodiment, the composition for promoting immune cell metabolism can be used for activating immunity, for example.

Thus, the composition according to the first embodiment of the present invention can be used for a composition for activating immunity. The composition for activating immunity containing the composition according to the first embodiment of the present invention is also encompassed by the present invention.

Hereinafter, the composition for activating immunity is also referred to as "the composition according to the second embodiment of the present invention".

The composition according to the first embodiment and the composition according to the second embodiment of the present invention each have an action to activate immunity and can be used to prevent or ameliorate a condition or disease caused by a decline in immune function.

Preventing a condition or disease encompasses preventing the onset, delaying the onset, reducing the incidence rate, reducing the onset risk, and the like. Ameliorating a condition or disease encompasses helping a subject recover from a condition or disease, alleviating a symptom of a condition or disease, favorably changing a symptom of a condition or disease, delaying or preventing the progress of a condition or disease, and the like.

Examples of the condition or disease caused by a decline in immune function include infectious diseases caused by microorganisms such as viruses and bacteria and various malignant tumors. Examples of the infectious diseases include infectious enteritis caused by bacteria such as cholera, enterotoxigenic Escherichia coli, Shigella, and Salmonella, and viruses through oral infection; influenza and common cold syndromes through respiratory tract infection; and stomatitis and periodontal disease through intraoral infection. Examples of the malignant tumors include epithelial malignant tumors that occur in solid organs such as the gastrointestinal tract, respiratory mucosa, liver, and kidney; and non-epithelial malignant tumors that occur in locomotor organs, soft tissues, and the like. Examples of the infectious diseases also include symptoms from a virus or the like, such as herpes virus, with which a subject has been infected in the past and which has been dormant in the body of the subject.

Preferably, the composition of the present invention is used to prevent or ameliorate at least one selected from the conditions or diseases described above.

Hereinafter, the composition for promoting immune cell metabolism of the present invention and the composition for activating immunity of the present invention are sometimes simply referred to as "the composition of the present invention" as a collective term.

The composition of the present invention is applicable for therapeutic use (medical use) and non-therapeutic use (non-medical use). The term "non-therapeutic" is a concept that does not include medical activities, i.e., a concept that does not include methods of surgery, therapy, or diagnosis of humans.

The composition of the present invention can be provided as an agent or the like, for example, but it is not limited thereto. The agent can be directly provided as a composition or can be provided as a composition containing the agent. In one embodiment, the composition for promoting immune cell metabolism of the present invention can also be referred to as an agent for promoting immune cell metabolism. In one embodiment, the composition for activating immunity of the present invention can also be referred to as an agent for activating immunity.

In order to sufficiently obtain the effect of the present invention, preferably, the composition of the present invention is an oral composition. The oral composition may be a food or beverage, an oral pharmaceutical product, an oral quasi-pharmaceutical product, or feed, preferably a food or beverage or an oral pharmaceutical product, more preferably a food or beverage.

The composition of the present invention may contain optional additives and optional components in addition to L-ergothioneine or a salt thereof, as long as the effect of the present invention is not impaired. Such additives and components can be selected according to the composition form or the like. Those that can be generally used in food and beverages, pharmaceutical products, quasi-pharmaceutical products, feed, and the like can be used. Preferably, the composition of the present invention does not contain Toll-like receptor ligands. When the composition of the present invention is provided as a food or beverage, a pharmaceutical product, a quasi-pharmaceutical product, feed, or the like, the production method is not limited, and any common method can be used for production.

For example, when the composition of the present invention is provided as a food or beverage, various types of food or beverages can be provided by adding a component usable in food and beverages (e.g., a food material or an optional food additive) to L-ergothioneine or a salt thereof. Non-limiting examples of the food or beverage include general food and beverages, health foods, health beverages, foods with function claims, foods for specified health uses, dietary supplements, and food and beverages for the sick. The health foods, foods with function claims, foods for specified health uses, dietary supplements, and the like can be used, for example, in various forms of preparations such as fine granules, tablets, granules, powders, capsules, chewable tablets, syrups, liquids, and liquid foods.

When the composition of the present invention is provided as a pharmaceutical product or a quasi-pharmaceutical product, for example, a pharmacologically acceptable carrier, an optional additive, or the like can be added to L-ergothioneine or a salt thereof to provide pharmaceutical products or quasi-pharmaceutical products in various dosage forms. Such a carrier, an additive, or the like may be any pharmacologically acceptable one that can be used in pharmaceutical products or quasi-pharmaceutical products. Examples thereof include excipients, binders, disintegrants, lubricants, antioxidants, and colorants. One or more of these can be used. The administration form of pharmaceutical products or quasi-pharmaceutical products may be oral or parenteral. Oral administration is preferred in order to obtain the effect of the present invention more sufficiently. When the composition of the present invention is provided as a pharmaceutical product or a quasi-pharmaceutical product, preferably, it is an oral pharmaceutical product or an oral quasi-pharmaceutical product. Examples of dosage forms for oral administration include liquids, tablets, powders, fine granules, granules, sugar-coated tablets, capsules, suspensions, emulsions, and chewable tablets. Examples of dosage forms for parenteral administration include injection and infusion. The pharmaceutical product and the quasi-pharmaceutical product may be for non-human animals.

When the composition of the present invention is provided as feed, L-ergothioneine or a salt thereof is simply added to feed. The feed includes feed additives. Examples of the feed include livestock feed for animals such as cows, pigs, chickens, sheep, and horses; feed for small animals such as rabbits, rats, and mice; and pet food for animals such as dogs, cats, and birds.

The amount of L-ergothioneine or a salt thereof in the composition of the present invention is not limited and can be set according to the composition form and the like.

The amount of L-ergothioneine or a salt thereof in the composition of the present invention is, for example, preferably 0.0001 wt% or more, more preferably 0.001 wt% or more and is preferably 90 wt% or less, more preferably 50 wt% or less in terms of L-ergothioneine. In one embodiment, the amount of L-ergothioneine or a salt thereof in the composition is preferably 0.0001 to 90 wt%, more preferably 0.001 to 50 wt% in terms of L-ergothioneine. In one embodiment, when the composition of the present invention is provided as a food or beverage, a pharmaceutical product, a quasi-pharmaceutical product, feed, or the like, preferably, the amount of L-ergothioneine or a salt thereof is in the above ranges.

In the case of L-ergothioneine, the expression "the amount in terms of ergothioneine" or a similar expression refers to the amount of L-ergothioneine. In the case of a salt of L-ergothioneine, the expression refers to a value obtained by multiplying the molar number of the salt by the molecular weight of L-ergothioneine.

The composition of the present invention can be fed or administered by a method appropriate to the composition form. In order to more sufficiently obtain the effect of the present invention, preferably, the composition of the present invention is orally fed (orally administered). The intake (administration amount) of the composition of the present invention is not limited as long as it is an amount that provides an effect of promoting immune cell metabolism or an amount that provides an effect of activating immune function. The intake may be appropriately set according to the administration form, administration method, body weight of a subject, and the like.

In one embodiment, when orally feeding or administering the composition of the present invention to a human (adult) as a subject, the intake of L-ergothioneine or a salt thereof is preferably 2 mg or more, more preferably 5 mg or more, still more preferably 10 mg or more, and is preferably 50 mg or less, more preferably 25 mg or less, still more preferably 20 mg or less in terms of L-ergothioneine per day. In one embodiment, when orally feeding or administering to a human (adult) as a subject, the intake of L-ergothioneine or a salt thereof is preferably 2 to 50 mg, more preferably 5 to 25 mg, still more preferably 5 to 20 mg, particularly preferably 10 to 20 mg in terms of L-ergothioneine per day. Preferably, the above amount of the composition is fed or administered in one or more portions per day, for example, in one portion or several portions (for example, two or three portions) per day. In one embodiment of the present invention, the composition of the present invention may be an oral composition for feeding or administering a human with the above amount of L-ergothioneine or a salt thereof per 60 kg body weight per day.

In one embodiment, when parenterally administering the composition of the present invention to a human (adult), the administration amount of L-ergothioneine or a salt thereof is, for example, preferably 2 to 50 mg, more preferably 5 to 25 mg, still more preferably 5 to 20 mg, particularly preferably 10 to 20 mg in terms of L-ergothioneine per day.

In one embodiment, in the case of a human (adult), preferably, the above amount of L-ergothioneine or a salt thereof is fed or administered per 60 kg body weight per day.

In one embodiment, preferably, the amount of L-ergothioneine or a salt thereof in the composition of the present invention per adult daily intake is 2 to 50 mg in terms of L-ergothioneine. The amount of L-ergothioneine or a salt thereof in the composition of the present invention per adult daily intake is more preferably 5 to 25 mg, still more preferably 5 to 20 mg, particularly preferably 10 to 20 mg in terms of L-ergothioneine.

Continuous feeding or administration of L-ergothioneine or a salt thereof is likely to result in a higher effect of promoting immune cell metabolism. Continuous feeding or administration of L-ergothioneine or a salt thereof is likely to result in a higher effect of activating immunity.

Thus, in a preferred embodiment, the composition of the present invention is continuously fed or administered. In one embodiment of the present invention, the composition of the present invention is continuously fed or administered preferably for at least one week, more preferably for at least two weeks.

The composition of the present invention may be fed or administered to any subject (subject to administration). The subject is preferably a human or non-human mammal, more preferably a human.

The composition of the present invention may be fed or administered to a subject needing or wanting to promote immune cell metabolism and a subject needing or wanting to activate immunity. In one embodiment, the subject to administration may be one with a condition or disease caused by a decline in immune function. In one embodiment, the subject to administration of the composition of the present invention may be a healthy person. The subject to administration of the composition of the present invention may also be a healthy person whose immune function of immune cells has declined with aging.

The composition of the present invention may be labeled with a function claim based on the promotion of immune cell metabolism and/or a function claim based on the activation of immune function. For example, the composition of the present invention may be labeled with one or more function claims such as "promoting immune cell metabolism", "activating mitochondrial function", and "activating immunity".

In one embodiment of the present invention, preferably, the composition of the present invention is a food or beverage labeled with such a function claim. The label may be one indicating use of the composition to obtain the function. The label may be directly attached to the composition or may be attached to a container or a package of the composition.

The present invention encompasses the following methods and uses:
a method of promoting immune cell metabolism, including feeding or administering L-ergothioneine or a salt thereof;
a method of promoting immune cell metabolism by activating mitochondrial function, including feeding or administering L-ergothioneine or a salt thereof;
a method of activating immunity, including feeding or administering L-ergothioneine or a salt thereof;
use of L-ergothioneine or a salt thereof to promote immune cell metabolism;
use of L-ergothioneine or a salt thereof to promote immune cell metabolism by activating mitochondrial function; and
use of L-ergothioneine or a salt thereof to activate immunity.

Feeding or administering L-ergothioneine or a salt thereof to a subject can promote immune cell metabolism or can promote immune cell metabolism by an effect of activating mitochondrial function, thereby providing the effect of activating immunity. In one embodiment, the method of promoting immune cell metabolism can be used to activate immunity, for example. Preferably, L-ergothioneine or a salt thereof is orally fed or administered.

The methods may be therapeutic or non-therapeutic. The uses may be therapeutic or non-therapeutic.

In the methods and uses, L-ergothioneine or a salt thereof, preferred embodiments thereof, and the like are as described above for the composition of the present invention. In the methods and uses, preferably, L-ergothioneine or a salt thereof is fed or administered to a subject one or more times per day, for example, one to several times (e.g., two to three times) per day. The uses are preferably for humans or non-human mammals, more preferably for humans. In one embodiment, L-ergothioneine or a salt thereof can be used to obtain the effect of activating immunity by promoting immune cell metabolism or by promoting immune cell metabolism via activation of mitochondrial function.

In the methods and uses, L-ergothioneine or a salt thereof is simply used in an amount (effective amount) that provides the effect of promoting immune cell metabolism.

In the methods and uses, L-ergothioneine or a salt thereof is simply used in an amount (effective amount) that provides the effect of activating mitochondrial function.

In the methods and uses, L-ergothioneine or a salt thereof is simply used in an amount (effective amount) that provides the effect of activating immunity.

L-ergothioneine or a salt thereof, preferred administration amounts thereof, preferred administration subjects, and the like are as described above for the composition of the present invention. L-ergothioneine or a salt thereof may be directly fed or administered or may be fed or administered as a composition containing L-ergothioneine or a salt thereof. For example, the composition of the present invention may be fed or administered.

L-ergothioneine or a salt thereof can be used to produce a food or beverage, a pharmaceutical product, a quasi-pharmaceutical product, feed, or the like for use in promoting immune cell metabolism. In one embodiment, the present invention also encompasses use of L-ergothioneine or a salt thereof to produce a composition for promoting immune cell metabolism.

The present invention also encompasses L-ergothioneine or a salt thereof for use in promoting immune cell metabolism.

L-ergothioneine or a salt thereof can also be used to produce a food or beverage, a pharmaceutical product, a quasi-pharmaceutical product, feed, or the like for use in activating mitochondrial function. In one embodiment, the present invention also encompasses use of L-ergothioneine or a salt thereof to produce a composition for activating mitochondrial function.

The present invention also encompasses L-ergothioneine or a salt thereof for use in activating mitochondrial function.

L-ergothioneine or a salt thereof can be used to produce a food or beverage, a pharmaceutical product, a quasi-pharmaceutical product, feed, or the like for use in activating immunity. In one embodiment, the present invention also encompasses use of L-ergothioneine or a salt thereof to produce a composition for activating immunity.

The present invention also encompasses L-ergothioneine or a salt thereof for use in activating immunity.

### EXAMPLES

The present invention is described in further detail below with reference to an example, but the scope of the present invention is not limited thereto.

### <Example 1>

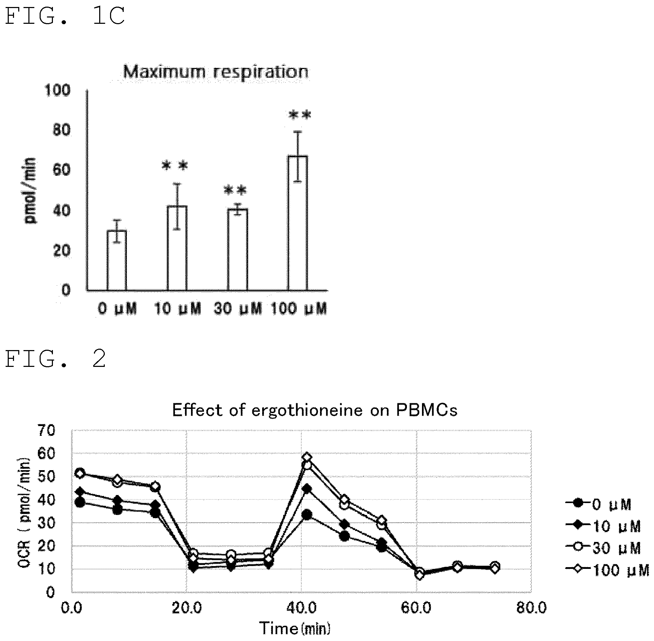

The effect of the compound (L-ergothioneine) of the present invention on mitochondrial function was analyzed. Ergothioneine was added to human peripheral blood mononuclear cells (PBMCs) under the following conditions. The extracellular oxygen level and pH were measured in real time using an XFe96 flux analyzer (available from Agilent Technologies). XFe technology uses solid-state sensors to simultaneously measure both oxygen consumption rate (OCR) and extracellular acidification rate (ECAR) to determine effects on oxidative phosphorylation (OXPHOS) and glycolysis simultaneously. FIG. 2 shows the results regarding the oxygen consumption rate (OCR) obtained.
Then, the cells were sequentially exposed to various mitochondrial function inhibitors to evaluate cell metabolism.

### (Conditions for adding ergothioneine to human PBMCs)

Human PBMCs were kept in an XF assay medium (XF RPMI1640 medium). Here, L-ergothioneine was adjusted to a final concentration of 0, 10, 30, or 100 µM. The cells (30,000 cells/50 µL/well) were seeded onto an XF96-well cell culture plate coated with PDL and were centrifuged at 200 g for one minute so that the cells were adhered to the plate. After culturing at 37°C in the absence of CO₂ for 20 minutes, the XF assay medium (130 µL) was added gently, followed by further culturing at 37°C in the absence of CO₂ for 40 minutes. The following mitochondrial stress test was performed on the obtained human PBMCs to which ergothioneine was added.

### <Mitochondrial stress test>

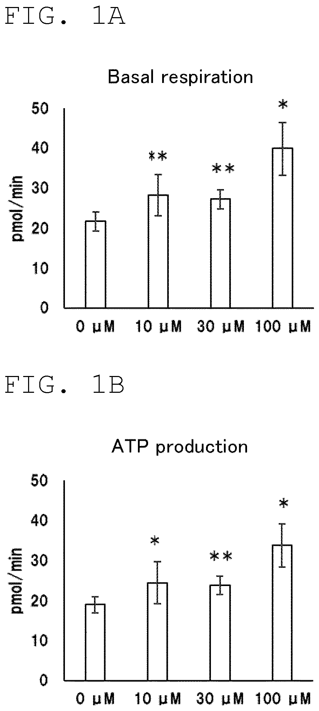

An XF Mit Stress Kit (available from Agilent Technologies) was used for the experiment. Compounds (1) to (3) described below were sequentially added to the obtained human PBMCs to which ergothioneine was added, and the OCR were measured, whereby parameters serving as major indices for evaluation of mitochondrial function, such as basal respiration, ATP production, and maximum respiration, were calculated. The results obtained are shown in FIG. 1A (basal respiration), FIG. 1B (ATP production), and FIG. 1C (maximum respiration) to facilitate understanding of a possible mechanism of mitochondria metabolism.

Compounds:
(1) 1 µM oligomycin
(2) 1 µM FCCP (carbonyl cyanide-p-trifluoromethoxyphenylhydrazone)
(3) 0.5 µM rotenone and antimycin A

The mitochondrial basal respiration, ATP production, and maximum respiration can be sequentially evaluated by sequentially adding the compounds (1) to (3) to the human PBMCs and measuring the OCR.

FIGS. 1A to 1C show the resulting values expressed as mean ± standard deviation. A significance test was performed by Dunnett's test (test by multiple comparison assuming equal variance, p-value) using an Excel analysis tool to obtain test results relative to the final concentration (0 µM) of ergothioneine (*: p < 0.05, **: p < 0.01) .

Oligomycin is a known ATP synthase inhibitor and prevents ATP formation. Oligomycin treatment enables measurement of the amount of oxygen consumption related to ATP production and ATP turnover. Adding oligomycin results in a decrease in OCR under normal conditions. The residual OCR is related to the natural proton leak.

FCCP is a protonophore and is a known uncoupler of oxygen consumption from ATP production. FCCP treatment allows the maximum achievable transfer of electrons and oxygen consumption rate and enables measurement of reserve capacity.

Rotenone and antimycin A are known inhibitors of complexes I and III of the electron transport chain, respectively. Treatment with these compounds inhibits electron transport completely, and any residual oxygen consumption is due to non-mitochondrial activity by oxygen dependent enzymes.

FIG. 1A is a graph showing the impact of ergothioneine on the OCR related to basal respiration. A significant increase in mitochondrial basal respiration can be confirmed in the human PBMCs to which ergothioneine was added at a final concentration of 10 µM, 30 µM, or 100 µM.

FIG. 1B is a graph showing the impact of ergothioneine on the OCR related to ATP-related respiration. A significant increase in mitochondrial ATP production can be confirmed in the human PBMCs to which ergothioneine was added at a final concentration of 10 µM, 30 µM, or 100 µM.

FIG. 1C is a graph showing the impact of ergothioneine on the OCR related to maximum respiration. A significant increase in mitochondrial maximum respiration can be confirmed in the human PBMCs to which ergothioneine was added at a final concentration of 10 µM, 30 µM, or 100 µM.

FIG. 2 is a graph showing the impact of ergothioneine on the oxygen consumption rate (OCR) of the human PBMCs over time. An increase in mitochondrial oxygen consumption rate can be confirmed in the human PBMCs to which ergothioneine was added at a final concentration of 10 µM, 30 µM, or 100 µM.

As described, the results in FIGS. 1A to 1C confirm that adding L-ergothioneine to the human PBMCs significantly increased their mitochondrial basal respiration, ATP production, and maximum respiration, showing that the mitochondrial function was activated and that the metabolism of the human PBMCs (immune cells) were promoted. The results in FIG. 2 confirm that adding L-ergothioneine to the human PBMCs improved the oxygen consumption rate (OCR) of the human PBMCs (immune cells) and promoted immune cell metabolism.

## Claims

1. A composition for promoting immune cell metabolism, comprising
L-ergothioneine or a salt thereof as an active ingredient.

2. The composition according to claim 1,
wherein the composition promotes immune cell metabolism by activating mitochondrial function.

3. A composition for activating immunity, comprising
the composition according to claim 1 or 2.

4. The composition according to any one of claims 1 to 3,
wherein the composition is an oral composition.

5. The composition according to any one of claims 1 to 4,
wherein the composition is a food or beverage.

6. The composition according to any one of claims 1 to 5,
wherein an amount of L-ergothioneine or a salt thereof in the composition per adult daily intake is 2 to 50 mg in terms of L-ergothioneine.

7. A method of promoting immune cell metabolism, comprising
administering L-ergothioneine or a salt thereof.

8. A method of activating immunity, comprising
administering L-ergothioneine or a salt thereof.

9. Use of L-ergothioneine or a salt thereof to promote immune cell metabolism.

10. Use of L-ergothioneine or a salt thereof to activate immunity.
